# EUROPEAN PATENT APPLICATION

(11) **EP 4 793 359 A1**
(43) Date of publication of application: **19.08.2026**
(21) Application number: 25158189.8
(22) Date of filing: 17.02.2025
(51) Int. Cl.: C12P 19/02, C12P 19/04, C12P 19/12

(54) **PRODUCTION OF CARBOHYDRATES BY KNALLGAS HYDROGENOPHILUS BACTERIA**

(71) Applicant: COLIPI GmbH, 21079 Hamburg (DE)
(72) Inventor: Agraval, Nisha, 21109 Hamburg (DE); de Witt, Jan Alexander, 21075 Hamburg (DE); Arbter, Philipp, 21109 Hamburg (DE)
(74) Representative: Eisenführ Speiser

(57) **Abstract**

The present invention relates to a method for producing one or more carbohydrate(s) by using a genetically modified cell. Further, the present invention relates to such a genetically modified cell and to the use of such a genetically modified cell for producing one or more carbohydrate(s), preferably in a method according to the invention. The present invention also relates to a mixture comprising such a genetically modified cell and one or more carbohydrate(s), which may be obtained by or obtainable by a method according to the invention.

## Description

The present invention relates to a method for producing one or more carbohydrate(s) by using a genetically modified cell. Further, the present invention relates to such a genetically modified cell and to the use of such a genetically modified cell for producing one or more carbohydrate(s), preferably in a method according to the invention. The present invention also relates to a mixture comprising such a genetically modified cell and one or more carbohydrate(s), which may be obtained by or obtainable by a method according to the invention.

Carbohydrates, particularly sugars such as glucose, fructose, sucrose, starch, and trehalose are essential molecules in the biochemical and biomedical industry and in our daily lives. Carbohydrates find numerous applications in industrial processes, in the production of nutritional products and pharmaceuticals. Thus, carbohydrates are ubiquitous molecules and need to be available in large quantities.

Currently, carbohydrates, particularly sugars such as glucose, fructose, sucrose, starch, and trehalose are predominantly derived from agricultural sources.

Glucose is primarily produced by enzymatic hydrolysis of corn starch or wheat starch. Fructose is derived from fruits, vegetables and by the enzymatic hydrolysis of starch and sucrose. Sucrose is primarily obtained from sugarcane and sugar beet. Starch is usually obtained from crops, such as corn, potatoes, wheat, rice, and cassava. Trehalose is found in honey and certain plants.

For providing such carbohydrates in the required amounts as described above, a large-scale cultivation of these plants is needed. Such a large-scale cultivation covers huge land areas and thus comes with significant environmental consequences.

The resource-intense nature of agriculture contributes to deforestation, habitat loss, and soil degradation due to monoculture practices. Moreover, crops like sugarcane require extensive water inputs, which exacerbates water scarcity in vulnerable regions and blocks masses of water, which could be used elsewhere. Furthermore, the agricultural sector is a notable source of greenhouse gas emissions, which contributes to global climate change.

Moreover, relying only on agricultural sources for providing carbohydrates bears a certain risk. Most of the cultivated plants are seasonal and can only be used for the production of carbohydrates during particular seasons. Moreover, since extreme weather events become more and more frequent, thus increasing the times of drought, fires and floods, the percentage of crop shortfall increases. Crop shortfall in turn causes a significant reduction of the starting materials for producing carbohydrates. Thus, when relying on agricultural sources for producing carbohydrates, a strong dependency on the particular season and on extreme weather events need to be considered.

Yet, with a growing global population and growing industry, the demands of carbohydrates persistently increase. There is thus a great need for providing alternatives.

Using bacterial strains for the production of carbohydrates, particularly sugars such as glucose, fructose, sucrose, starch, and trehalose, as an alternative to agricultural sources has been described in the art. In such methods, *Cupriavidus necator* or E. *coli* are commonly used as bacterial strains.

For example, WO 2021/158657 A1 describes an engineered *Cupriavidus necator* bacterium for producing biomolecules, including a sugar feedstock, which may comprise e.g. glucose, fructose, or sucrose.

With such approaches, carbohydrates may be produced without relying on agricultural sources. Consequently, these approaches do not or only marginally provide the environmental consequences described above. Further, without relying on agricultural sources, these approaches are not affected by the respective season or by extreme weather events. Thus, a continuous production can be achieved during the entire year, at any climate and with any weather event.

Additionally, such approaches require less water input than methods based on agricultural sources and do not lead to deforestation, habitat loss, and soil degradation.

Using bacterial strains for the production of carbohydrates is thus a promising approach for replacing at least a proportion of the current production of carbohydrates, which is mainly based on agricultural sources.

However, for providing a greater impact and for replacing larger proportions of the current production of carbohydrates, it is advantageous to further increase the production yields and/or the process parameters of such methods.

The primary object of the present invention was thus to provide a method for producing one or more carbohydrate(s), with which the disadvantages described above are reduced and/or with which an increased production yield is obtained.

The primary object of the present invention is solved by a method for producing one or more carbohydrate(s),
wherein the, one, two, three or all carbohydrate(s) is/are selected from the group consisting of glucose, fructose, trehalose, and starch,
the method comprising the steps

i) providing a genetically modified cell,
   wherein the cell is selected from the genus *Hydrogenophilus,* preferably of the species *Hydrogenophilus thermoluteolus,*
   wherein the genetic modification is or comprises
      a) a reduction of the Calvin-Benson cycle activity, and/or a reversion of the glyoxylate cycle activity, and/or an introduction of the reductive TCA cycle activity,
         and
      b.1) the heterologous expression of one, two, three or more or all gene(s) encoding an enzyme of group A,
         wherein the enzymes of group A are selected from the group consisting of glucose-1-phosphatase, glucose-6-phosphatase, alpha,alpha-trehalose phosphorylase, trehalase, Pase12, Pase-MG7, Pase15, MF2, and combinations thereof,
         and/or
      b.2) a deletion of or a reduction of the expression of one, two, three or more or all gene(s) encoding an enzyme of group B,
         wherein the enzymes of group B are selected from the group consisting of phosphopyruvate hydratase, phosphoglucomutase, glucose-1-phosphate uridylyltransferase, glucose-1-phosphate adenylyltransferase, glucose-6-phosphate isomerase, glycogen phosphorylase, glucokinase, 4-alpha-glucanotransferase, UTP glucose-1-phosphate uridylyltransferase, phosphoglycerate mutase, and combinations thereof,
ii) cultivating the cell provided in step i) at a temperature in the range of from 40 to 65 °C, preferably of from 42.5 to 62.5 °C, particularly preferably of from 45 to 60 °C, further preferably of from 47.5 to 57.5 °C, more preferably of from 50 to 55 °C,
   wherein cultivating includes providing CO₂, O₂ and H₂,
iii) purifying the produced carbohydrate(s) from the cell or cell culture cultivated in step ii).

It was surprisingly found that with the method according to the invention a higher yield of carbohydrates was obtained when compared to methods in the prior art, which use *Cupriavidus necator* or *E*. *coli.* Further, since the method according to the invention does not relate on agricultural sources for producing carbohydrates, the environmental consequences described herein can be avoided or at least be reduced.

It was also surprisingly found that with the present method, higher temperatures may be applied than with methods using *Cupriavidus necator* or *E*. *coli,* such that the risk of contaminations is drastically reduced.

It was also surprisingly found that with the method according to the invention, the yield may be even further increased, since cells of the genus *Hydrogenophilus,* particularly of the species *Hydrogenophilus thermoluteolus,* lack transporters for e.g. glucose, fructose, starch, trehalose, sucrose and other carbohydrates, and thus the carbohydrates can accumulate intracellularly. In this way, unregulated export of the produced carbohydrates can be avoided and the yield be increased.

Moreover, by applying cells of the genus *Hydrogenophilus,* particularly of the species *Hydrogenophilus thermoluteolus,* the production of carbohydrates only relies on CO₂ and H₂ and no additional organic substrates are required (as in heterotrophic systems).

It was also surprisingly found that with the method according to the invention, trehalose can by synthesized without stress induction, which is usually required in other systems and renders the other systems inefficient. Thus, the method according to the invention provides a higher efficiency than other systems.

It was further surprisingly found that with the method according to the invention, the yield of the produced carbohydrates, i.e. glucose, fructose, trehalose or starch, was increased by 20 - 50 % compared to similar approaches but using *Cupriavidus necator* or E. *coli.*

Even further, it was surprisingly found that with the engineered pathways in the cell, as described herein, the current bottlenecks (such as sucrose reuptake and inefficient flux distribution) in existing other microbial systems can be overcome or at least be reduced. Thus, limitations in the cell's metabolism can be overcome or at least be reduced to further increase the yield of the produced carbohydrates.

Preferably, the term "glucose" refers to D-glucose, preferably to α-D-glucose. Preferably, the term refers to α-D-Glucofuranose. Preferably, the term refers to β-D-Glucofuranose. Preferably, the term refers to α-D-Glucopyranose. Preferably, the term refers to β-D-Glucopyranose.

Preferably, the term "fructose" refers to D-fructose, preferably to α-D-fructose. Preferably, the term refers to α-D-Fructofuranose. Preferably, the term refers to β-D-Fructofuranose. Preferably, the term refers to α-D-Fructopyranose. Preferably, the term refers to β-D-Fructopyranose.

Preferably, the term "trehalose" refers to α-D-gluco-pyranosyl-(1→1)-α-D-gluco-pyra-noside.

Preferably, the term "starch" refers to a polysaccharide according to the formula (C₆H₁₀O₅)ₙ, comprising or consisting of α-D-Glucose-monomers. Preferably, the polysaccharide comprises or consists of amylose and amylopectin subunits.

Bacteria of the genus *Hydrogenophilus* are typically hydrogen-oxidizing bacteria. When considering the production of carbohydrates, these bacteria metabolically waste significant amounts of the (hydrogen-derived) energy to fuel respiration and to fix CO₂ by the Calvin-Benson cycle. Likewise, these bacteria metabolically waste significant amounts of energy to fuel the glyoxylate cycle. This results in lower product yields and the fact that hydrogen can account for up to 80% of production costs for *Hydrogenophilus-*driven bioprocesses.

Bacteria of the genus *Hydrogenophilus* do not conduct the reductive TCA cycle (reductive tricarboxylic acid cycle). It is estimated that using the reductive TCA cycle for fixing CO₂ to produce compounds such as acetate, would require up to 27 % less molecules H₂ than via the Calvin-Benson cycle. Thus, promoting the reductive TCA cycle would shift the production of carbohydrates to higher yields per consumed H₂ molecule. It was calculated that in case only the reductive TCA cycle instead of the Calvin-Benson cycle is applied for fixing CO₂, a yield increase of approx. 53 % can be expected for the production of glucose (mol glucose per mol consumed H₂). It was found that the heterologous expression of citryl-CoA synthetase (CCS) and/or citryl-CoA lyase (CCL) introduced the reductive TCA cycle in bacteria of the genus *Hydrogenophilus,* which may then be used for the production of carbohydrates with a higher yield, as described above.

Thus, it is very advantageous with the method according to the invention that the genetic modification comprises a reduction of the Calvin-Benson cycle activity, and/or a reversion of the glyoxylate cycle activity, and/or an introduction of the reductive TCA cycle activity. With such a modification, the production yields are further increased.

Preferably, the term "a reduction of the Calvin-Benson cycle activity" refers to a reduction or (complete) inhibition of the metabolic activity of the Calvin-Benson cycle, which may be observed by reduced amounts of the respective product(s) of the cycle. Particularly preferably, the term refers to a reduction in the enzyme activity of one, two, three or more or all enzymes of the Calvin-Benson cycle, thus the term preferably refers to a reduction of the Calvin-Benson cycle enzymatic activity. Such a reduction can be obtained by reducing the expression of one or more genes encoding one, two, three or more or all enzymes of the Calvin-Benson cycle and/or by increasing the expression of one or more genes encoding one, two, three or more inhibitors of at least one enzyme of the Calvin-Benson cycle.

Preferably, the term "a reversion of the glyoxylate cycle activity" describes that the glyoxylate cycle runs in reverse order. Thus, preferably, instead of producing succinate from acetyl-CoA and glyoxylate, acetyl-CoA is produced from succinate, glyoxylate, isocitrate, citrate, oxaloacetate and/or malate. Within the glyoxylate cycle (in natural order), malate is produced from glyoxylate and acetyl-CoA, however, the respective enzymes are generally able to catalyse the reactions in both directions. For example by the heterologous expression of ATP-dependent malate thiokinase and/or a malyl-CoA lyase, malate is excessively converted to acetyl-CoA (i.e. in reverse order compared to the natural glyoxylate cycle), which in turn forces the enzymes of the glyoxylate cycle to catalyse the enzymatic reactions of the glyoxylate cycle in reverse direction and thus, the glyoxylate cycle is reversed.

Preferably, the term "introduction of the reductive TCA cycle activity" refers to a promotion of the metabolic activity of the reductive TCA cycle in the respective cell, which may be observed by detecting the respective product(s) of the cycle. Particularly preferably, the term refers to an introduction or increase of the enzyme activity of one, two, three or more or all enzymes of the reductive TCA cycle, thus the term preferably refers to an introduction or increase of the reductive TCA cycle enzymatic activity. Such an introduction can be obtained by the (heterologous) expression of one or more genes encoding one, two, three or more or all enzymes of the reductive TCA cycle, particularly preferably by the heterologous expression of citryl-CoA synthetase (CCS) and/or citryl-CoA lyase (CCL), preferably citryl-CoA synthetase (CCS) and/or citryl-CoA lyase (CCL) from *Hydrogenobacter thermophilus.*

Typically, the genetic modification may be obtained by any means, such as deletion, insertion or replacement of nucleotides in a gene or one or more genes of interest and/or by transfecting a vector comprising a gene of interest and/or by increasing or reducing or activating or inhibiting a transcription or translation factor.

It is preferred in the method according to the invention that the reduction of the Calvin-Benson cycle activity is represented or achieved by
i.a.1) the deletion or a reduction of the homologous expression of ribulose-1,5-bisphos-phat-carboxylase/-oxygenase.

It is preferred in the method according to the invention that the introduction of the reductive TCA cycle activity is represented or achieved by
i.a.1) the deletion or a reduction of the homologous expression of ribulose-1,5-bisphos-phat-carboxylase/-oxygenase, and/or
i.a.2) an increase of the expression of citrate synthase, and/or
i.a.3) the heterologous expression of citryl-CoA synthetase (CCS) and/or citryl-CoA lyase (CCL), preferably citryl-CoA synthetase (CCS) and/or citryl-CoA lyase (CCL) from *Hydrogenobacter thermophilus,* and/or
i.a.4) the heterologous expression of pyruvate carboxylase and/or pyruvate synthase and/or alpha-ketoglutarate synthase, preferably pyruvate carboxylase and/or pyruvate synthase and/or alpha-ketoglutarate synthase from *Hydrogenobacter thermophilus,* and/or
i.a.5) the heterologous expression of ATP-dependent malate thiokinase and/or a malyl-CoA lyase.

It is particularly preferred in the method according to the invention that the introduction of the reductive TCA cycle activity is represented or achieved by
i.a.3) the heterologous expression of citryl-CoA synthetase (CCS) and/or citryl-CoA lyase (CCL), preferably citryl-CoA synthetase (CCS) and/or citryl-CoA lyase (CCL) from *Hydrogenobacter thermophilus.*

It is preferred in the method according to the invention that the reversion of the glyoxylate cycle activity is represented or achieved by
i.a.5) the heterologous expression of ATP-dependent malate thiokinase and/or a malyl-CoA lyase.

Preferably, the term "deletion of one, two, three or more or all gene(s)" includes the deletion of the entire gene(s) but also the deletion of a part of the gene(s), provided that the expression of the gene(s) and/or the activity of the encoded enzyme(s) is reduced or absent.

Preferably, the term "reduction of the expression of one, two, three or more or all gene(s)" includes reducing the amount or activity of one or more transcription or translation factor(s) of the gene(s).

Preferably, the term "glucose-1-phosphatase" refers to an enzyme classified according to the Enzyme Commission number (EC number), with the EC class EC 3.1.3.10.

Preferably, the term "glucose-6-phosphatase" refers to an enzyme classified according to the Enzyme Commission number (EC number), with the EC class EC 3.1.3.9.

Preferably, the term "alpha,alpha-trehalose phosphorylase" refers to an enzyme classified according to the Enzyme Commission number (EC number), with the EC class EC 2.4.1.64.

Preferably, the term "trehalase" refers to an enzyme classified according to the Enzyme Commission number (EC number), with the EC class EC 3.2.1.28.

Preferably, the term "Pase12" refers to the Pase12 phosphatase from *Thermoclostridium stercorarium,* preferably as described in Tian C, Yang J, Liu C, Chen P, Zhang T, Men Y, Ma H, Sun Y, Ma Y. Engineering substrate specificity of HAD phosphatases and multienzyme systems development for the thermodynamic-driven manufacturing sugars. Nat Commun. 2022 Jun 23;13(1):3582. doi: 10.1038/s41467-022-31371-8. PMID: 35739124; PMCID: PMC9226320.

Preferably, the term "Pase-MG7" refers to the Pase-MG7 phosphatase mutant, preferably the Y122E/L9M/E183D/H125I/F178L/S177T/Y181M mutant, preferably as described in Tian C, Yang J, Liu C, Chen P, Zhang T, Men Y, Ma H, Sun Y, Ma Y. Engineering substrate specificity of HAD phosphatases and multienzyme systems development for the thermodynamic-driven manufacturing sugars. Nat Commun. 2022 Jun 23;13(1):3582. doi: 10.1038/s41467-022-31371-8. PMID: 35739124; PMCID: PMC9226320.

Preferably, the term "Pase15" refers to the Pase15 phosphatase from *Thermoclostridium caenicola,* preferably as described in Tian C, Yang J, Liu C, Chen P, Zhang T, Men Y, Ma H, Sun Y, Ma Y. Engineering sub-strate specificity of HAD phosphatases and multienzyme systems development for the thermodynamic-driven manufacturing sugars. Nat Commun. 2022 Jun 23;13(1):3582. doi: 10.1038/s41467-022-31371-8. PMID: 35739124; PMCID: PMC9226320.

Preferably, the term "MF2" refers to the MF2 phosphatase mutant, preferably the S126F/Y181H mutant, preferably as described in Tian C, Yang J, Liu C, Chen P, Zhang T, Men Y, Ma H, Sun Y, Ma Y. Engineering substrate specificity of HAD phosphatases and multienzyme systems development for the thermodynamic-driven manufacturing sugars. Nat Commun. 2022 Jun 23;13(1):3582. doi: 10.1038/s41467-022-31371-8. PMID: 35739124; PMCID: PMC9226320.

Preferably, the term "phosphopyruvate hydratase" refers to an enzyme classified according to the Enzyme Commission number (EC number), with the EC class EC 4.2.1.11.

Preferably, the term "phosphoglucomutase" refers to an enzyme classified according to the Enzyme Commission number (EC number), with the EC class EC 5.4.2.2.

Preferably, the term "glucose-1-phosphate uridylyltransferase" refers to an enzyme classified according to the Enzyme Commission number (EC number), with the EC class EC 2.7.7.9.

Preferably, the term "glucose-1-phosphate adenylyltransferase" refers to an enzyme classified according to the Enzyme Commission number (EC number), with the EC class EC 2.7.7.27.

Preferably, the term "glucose-6-phosphate isomerase" refers to an enzyme classified according to the Enzyme Commission number (EC number), with the EC class EC 5.3.1.9.

Preferably, the term "glycogen phosphorylase" refers to an enzyme classified according to the Enzyme Commission number (EC number), with the EC class EC 2.4.1.1.

Preferably, the term "glucokinase" refers to an enzyme classified according to the Enzyme Commission number (EC number), with the EC class EC 2.7.1.2.

Preferably, the term "4-alpha-glucanotransferase" refers to an enzyme classified according to the Enzyme Commission number (EC number), with the EC class EC 2.4.1.25.

Preferably, the term "UTP glucose-1-phosphate uridylyltransferase" refers to an enzyme classified according to the Enzyme Commission number (EC number), with the EC class EC 2.7.7.9.

Preferably, the term "phosphoglycerate mutase" refers to an enzyme classified according to the Enzyme Commission number (EC number), with the EC class EC 5.4.2.11.

Preferably, the term "wherein cultivating includes providing CO₂, O₂ and H₂" refers to providing CO₂, O₂ and H₂ in gaseous form and typically refers to maintaining a certain concentration of CO₂, O₂ and H₂ in the gaseous medium in the environment of the cultivated cell. Usually, an incubator is used for cultivating the cell. In such a case, for example, the incubator maintains a certain concentration of CO₂, O₂ and H₂ in its interior. Preferably, the CO₂ concentration in the environment (e.g. within the incubator) is substantially maintained in the range of from 1 to 25 vol.-%, preferably of from 2 to 20 vol.-%, particularly preferably of from 5 to 15 vol.-%, further preferably of from 7.5 to 12.5 vol.-%. Preferably, the O₂ concentration in the environment (e.g. within the incubator) is substantially maintained in the range of from 0.5 to 15 vol.-%, preferably of from 1 to 10 vol.-%, particularly preferably of from 2.5 to 7.5 vol.-%. Preferably, the H₂ concentration in the environment (e.g. within the incubator) is substantially maintained in the range of from 60 to 97.5 vol.-%, preferably of from 70 to 95 vol.-%, particularly preferably of from 75 to 92.5 vol.-%, further preferably of from 80 to 90 vol.-%, especially preferably of from 82.5 to 87.5 vol.-%.

Preferably, for the cultivation, the cell is surrounded or in contact with a medium providing nutrition and metabolites for the cell. Typically, for the cultivation, any medium may be applied, preferably a minimal medium (such as minimal essential medium, e.g. MEM) is applied. Usually, the (minimal) medium is selected based on the particular cultivated cell. (Minimal) media for cultivating a cell of the genus *Hydrogenophilus* are commercially available. Preferably, the medium is selected such that the cell is maintained alive and preferably cell growth and proliferation are promoted.

Preferably, the term "cultivating" or "cultivation" refers to maintaining a cell alive and preferably to promoting growth and proliferation of the cell.

It is preferred in the method according to the invention that the cultivation in step ii) is applied for at least 2 h, preferably for at least 4 h, particularly preferably for at least 6 h, further preferably for at least 8 h.

It is further preferred in the method according to the invention that the cultivation in step ii) is applied for a time in the range of from 2 to 48 h, preferably of from 4 to 36 h, particularly preferably of from 6 to 24 h, further preferably of from 8 to 12 h.

The term "purifying" includes any method for physical separation of a chemical substance of interest (or at least increasing its concentration) from foreign or contaminating substances. The term includes but is not restricted to an isolation of said chemical substance of interest. Methods for purifying include centrifugation, chromatography such as column chromatography, extraction with a particular solvent or mixture of solvents, filtration, evaporation, distillation or crystallization, as well as any other methods known to a skilled person.

It was found that cells of the genus *Hydrogenophilus,* particularly cells of the species *Hydrogenophilus thermoluteolus,* utilize the CO₂ of their environment as carbon source and convert it to glycerate-3-phosphate. Starting from glycerate-3-phosphate, several conversions are possible and applied by cells of the genus *Hydrogenophilus,* particularly cells of the species *Hydrogenophilus thermoluteolus,* which may be used and modified for producing carbohydrates as described herein.

By their own enzymes, the cells convert glycerate-3-phosphate to D-fructose-6-phosphate (e.g. via phosphoglycerate kinase to glycerate-1,3-bisphosphate, subsequently via glyceraldehyde-3-phosphate dehydrogenase to glyceraldehyde-3-phosphate, subsequently via fructose-bisphosphate aldolase to D-fructose-1,6-bisphosphate and subsequently via phosphoglycerate kinase to D-fructose-6-phosphate).

Preferably, the term "phosphoglycerate kinase" refers to an enzyme classified according to the Enzyme Commission number (EC number), with the EC class EC 2.7.2.3.

Preferably, the term "glyceraldehyde-3-phosphate dehydrogenase" refers to an enzyme classified according to the Enzyme Commission number (EC number), with the EC class EC 1.2.1.12.

Preferably, the term "fructose-bisphosphate aldolase" refers to an enzyme classified according to the Enzyme Commission number (EC number), with the EC class EC 4.1.2.13.

To advantageously promote the conversion of glycerate-3-phosphate to D-fructose-6-phosphate, alternative conversions of glycerate-3-phosphate such as to glycerate-2-phosphate may be reduced or inhibited by a deletion of or a reduction of the expression of phosphoglycerate mutase (which would promote the conversion to glycerate-2-phosphate).

Additionally or alternatively, to advantageously promote the conversion of glycerate-3-phosphate to D-fructose-6-phosphate, alternative conversions of glycerate-3-phosphate such as to phosphoenolpyruvate (via glycerate-2-phosphate) may be reduced or inhibited by a deletion of or a reduction of the expression of phosphopyruvate hydratase (which would promote the conversion of glycerate-2-phosphate to Phosphoenolpyruvate).

Thus, the amount of glycerate-3-phosphate, which may be converted to D-fructose-6-phosphate may be increased, which in turn may increase the production of the carbohydrates as described herein.

For the production of glucose, the obtained D-fructose-6-phosphate may be converted to α-D-glucose-6-phosphate such as by the cells' own enzyme (e.g. glucose-6-phosphate isomerase). The obtained α-D-glucose-6-phosphate may then be converted to D-glucose by the heterologous expression of glucose-6-phosphatase and/or Pase 12, and/or Pase-MG7.

Optionally, the conversion of the obtained D-glucose back to α-D-glucose-6-phosphate may be reduced or inhibited by a deletion of or a reduction of the expression of glucokinase, to increase the yield of D-glucose.

Optionally, the conversion of the obtained α-D-glucose-6-phosphate to α-D-glucose-1-phosphate may be reduced or inhibited by a deletion of or a reduction of the expression of phosphoglucomutase, to promote the conversion to D-glucose as described above. However, as will be described below, this is optional, since α-D-glucose-1-phosphate may be converted to glucose as well.

Additionally or alternatively, the obtained α-D-glucose-6-phosphate may be converted to α-D-glucose-1-phosphate such as by the cells' own enzyme (e.g. phosphoglucomutase). The obtained α-D-glucose-1-phosphate may then be converted to D-glucose by the heterologous expression of glucose-1-phosphatase.

Optionally, the conversion of the obtained α-D-glucose-1-phosphate to UDP glucose may be reduced or inhibited by a deletion of or a reduction of the expression of glucose-1-phosphate uridylyltransferase, to promote the conversion to D-glucose described above. However, as will be described below, this is optional.

Additionally or alternatively, the obtained α-D-glucose-1-phosphate may be converted to trehalose such as by the cells' own enzymes (e.g. via UTP glucose-1-phosphate uridylyltransferase to UDP glucose, subsequently via trehalose 6-phosphate synthase to trehalose-6-phosphate and subsequently via trehalose-6-phosphatase to trehalose). The obtained trehalose may then be converted to D-glucose by the heterologous expression of e.g. alpha,alpha-trehalose phosphorylase and/or trehalase.

Preferably, the term "trehalose 6-phosphate synthase" refers to an enzyme classified according to the Enzyme Commission number (EC number), with the EC class EC 2.4.1.15.

Preferably, the term "trehalose-6-phosphatase" refers to an enzyme classified according to the Enzyme Commission number (EC number), with the EC class EC 3.1.3.12.

Optionally, the conversion of the obtained α-D-glucose-1-phosphate to ADP glucose, which does not lead to the production of glucose, may be reduced or inhibited by a deletion of or a reduction of the expression of glucose-1-phosphate adenylyltransferase.

For the production of fructose, the obtained D-fructose-6-phosphate may be converted to D-fructose by the heterologous expression of e.g. Pase15 and/or MF2.

Optionally, the conversion of the obtained D-fructose-6-phosphate to α-D-glucose-6-phosphate may be reduced or inhibited by a deletion of or a reduction of the expression of glucose-6-phosphate isomerase and/or the conversion of Glycerate-3P to Glycerate-2P may be reduced or inhibited by a deletion of or a reduction of the expression of phosphoglycerate mutase, to increase the yield of D-fructose.

For the production of trehalose, the obtained D-fructose-6-phosphate may be converted to trehalose such as by the cells' own enzymes (e.g. via α-D-glucose-6-phosphate, α-D-glucose-1-phosphate, UDP glucose, and trehalose-6-phosphate, such as with the enzymes as described above).

Optionally, the conversion of the obtained α-D-glucose-1-phosphate to ADP glucose, which does not lead to the production of trehalose, may be reduced or inhibited by a deletion of or a reduction of the expression of glucose-1-phosphate adenylyltransferase.

For the production of starch, the obtained D-fructose-6-phosphate may be converted to starch such as by the cells' own enzymes (e.g. via glucose-6-phosphate isomerase to α-D-glucose-6-phosphate, subsequently via phosphoglucomutase to α-D-glucose-1-phosphate, subsequently via glucose-1-phosphate adenylyltransferase to ADP glucose, subsequently via glycogen synthase to amylose, and subsequently via 1,4-alpha-glucan branching enzyme to starch).

Preferably, the term "glycogen synthase" refers to an enzyme classified according to the Enzyme Commission number (EC number), with the EC class EC 2.4.1.21.

Preferably, the term "1,4-alpha-glucan branching enzyme" refers to an enzyme classified according to the Enzyme Commission number (EC number), with the EC class EC 2.4.1.18.

Optionally, the conversion of the obtained starch to α-D-glucose-1-phosphate may be reduced or inhibited by a deletion of or a reduction of the expression of glycogen phosphorylase and/or 4-alpha-glucanotransferase, to increase the yield of starch.

Optionally, the conversion of the obtained α-D-glucose-1-phosphate to UDP glucose may be reduced or inhibited by a deletion of or a reduction of the expression of glucose-1-phosphate uridylyltransferase and/or UTP glucose-1-phosphate uridylyltransferase, to promote the conversion to starch described above.

It is preferred in the method according to the invention that the genetic modification of the cell provided in step i) comprises
b.1) the heterologous expression of one or more genes encoding glucose-1-phosphatase, glucose-6-phosphatase, alpha,alpha-trehalose phosphorylase, trehalase, Pase12, and/or Pase-MG7, and/or (preferably: and optionally),
b.2) a deletion of or a reduction of the expression of one or more genes encoding phosphoglycerate mutase, phosphopyruvate hydratase, phosphoglucomutase, glucose-1-phosphate uridylyltransferase, glucose-1-phosphate adenylyltransferase, and/or glucokinase,
preferably wherein the or one carbohydrate produced with the method is glucose.

Additionally or alternatively, it is preferred in the method according to the invention that the genetic modification of the cell provided in step i) comprises
b.1) the heterologous expression of one or more genes encoding Pase15, and/or MF2, and/or (preferably: and optionally),
b.2) a deletion of or a reduction of the expression of one or more genes encoding phosphoglycerate mutase, phosphopyruvate hydratase, and/or glucose-6-phosphate isomerase,
preferably wherein the or one carbohydrate produced with the method is fructose.

Additionally or alternatively, it is preferred in the method according to the invention that the genetic modification of the cell provided in step i) comprises
b.2) a deletion of or a reduction of the expression of one or more genes encoding phosphoglycerate mutase, phosphopyruvate hydratase, and/or glucose-1-phosphate adenylyltransferase,
preferably wherein the or one carbohydrate produced with the method is trehalose.

Additionally or alternatively, it is preferred in the method according to the invention that the genetic modification of the cell provided in step i) comprises
b.2) a deletion of or a reduction of the expression of one or more genes encoding phosphoglycerate mutase, phosphopyruvate hydratase, 4-alpha-glucanotransferase, UTP glucose-1-phosphate uridylyltransferase, glucokinase, phosphoglucomutase, and/or glycogen phosphorylase,
preferably wherein the or one carbohydrate produced with the method is starch.

Additionally or alternatively, it is preferred that one or more of the cell's own enzyme(s), each as described in the pathways above, may additionally be heterologously expressed in the cell, wherein the term "cell's own enzyme" refers to the enzyme function and does not need to be the enzyme of the respective cell or cell type. E.g. the phosphoglycerate kinase (which is also expressed in cells of the genus *Hydrogenophilus)* may additionally be expressed homologously in the method according to the invention, wherein the homologously expressed phosphoglycerate kinase may be a phosphoglycerate kinase of the same or of a different genus or, respectively, species.

It is thus preferred that the genetic mutation of the cell provided in step i) further comprises
c) the heterologous expression of one or more genes encoding phosphoglycerate kinase, glyceraldehyde-3-phosphate dehydrogenase, fructose-bisphosphate aldolase, phosphoglycerate kinase, glucose-6-phosphate isomerase, phosphoglucomutase, UTP glucose-1-phosphate uridylyltransferase, trehalose 6-phosphate synthase, trehalose-6-phosphatase, glucose-6-phosphate isomerase, phosphoglucomutase, glucose-1-phosphate adenylyltransferase, glycogen synthase, 1,4-alpha-glucan branching enzyme,
preferably of at least phosphoglycerate kinase,
preferably of at least glyceraldehyde-3-phosphate dehydrogenase,
preferably of at least fructose-bisphosphate aldolase,
preferably of at least phosphoglycerate kinase,
preferably of at least glucose-6-phosphate isomerase,
preferably of at least phosphoglucomutase,
preferably of at least UTP glucose-1-phosphate uridylyltransferase,
preferably of at least trehalose 6-phosphate synthase,
preferably of at least trehalose-6-phosphatase,
preferably of at least glucose-6-phosphate isomerase,
preferably of at least phosphoglucomutase,
preferably of at least glucose-1-phosphate adenylyltransferase,
preferably of at least glycogen synthase,
preferably of at least 1,4-alpha-glucan branching enzyme.

The present invention further relates to a genetically modified cell,
wherein the cell is selected from the genus *Hydrogenophilus,* preferably of the species *Hydrogenophilus thermoluteolus,*
wherein the genetic modification is or comprises
   a) a reduction of the Calvin-Benson cycle activity and/or a reversion of the glyoxylate cycle activity, and/or an introduction of the reductive TCA cycle activity,
      and
   b.1) the heterologous expression of one, two, three or more or all gene(s) encoding an enzyme of group A,
      wherein the enzymes of group A are selected from the group consisting of glucose-1-phosphatase, glucose-6-phosphatase, alpha,alpha-trehalose phosphorylase, trehalase, Pase12, Pase-MG7, Pase15, MF2, and combinations thereof, and/or
   b.2) a deletion of or a reduction of the expression of one, two, three or more or all gene(s) encoding an enzyme of group B,
      wherein the enzymes of group B are selected from the group consisting of phosphopyruvate hydratase, phosphoglucomutase, glucose-1-phosphate uridylyltransferase, glucose-1-phosphate adenylyltransferase, glucose-6-phosphate isomerase, glycogen phosphorylase, glucokinase, 4-alpha-glucanotransferase, UTP glucose-1-phosphate uridylyltransferase, phosphoglycerate mutase, and combinations thereof.

What is said herein with regard to the method according to the invention, applies accordingly to the genetically modified cell according to the invention.

Particularly what is said herein with regard to the genetic modification in light of the method according to the invention, applies accordingly to the genetic modification in light of the genetically modified cell according to the invention.

It is preferred for the genetically modified cell according to the invention that the reduction of the Calvin-Benson cycle activity is represented or achieved by
i.a.1) the deletion or a reduction of the homologous expression of ribulose-1,5-bisphos-phat-carboxylase/-oxygenase.

It is preferred in the method according to the invention that the introduction of the reductive TCA cycle activity is represented or achieved by
i.a.1) the deletion or a reduction of the homologous expression of ribulose-1,5-bisphos-phat-carboxylase/-oxygenase, and/or
i.a.2) an increase of the expression of citrate synthase, and/or
i.a.3) the heterologous expression of citryl-CoA synthetase (CCS) and/or citryl-CoA lyase (CCL), preferably citryl-CoA synthetase (CCS) and/or citryl-CoA lyase (CCL) from *Hydrogenobacter thermophilus,* and/or
i.a.4) the heterologous expression of pyruvate carboxylase and/or pyruvate synthase and/or alpha-ketoglutarate synthase, preferably pyruvate carboxylase and/or pyruvate synthase and/or alpha-ketoglutarate synthase from *Hydrogenobacter thermophilus,* and/or
i.a.5) the heterologous expression of ATP-dependent malate thiokinase and/or a malyl-CoA lyase.

It is particularly preferred in the method according to the invention that the introduction of the reductive TCA cycle activity is represented or achieved by
i.a.3) the heterologous expression of citryl-CoA synthetase (CCS) and/or citryl-CoA lyase (CCL), preferably citryl-CoA synthetase (CCS) and/or citryl-CoA lyase (CCL) from *Hydrogenobacter thermophilus.*

It is preferred for the genetically modified cell according to the invention that the reversion of the glyoxylate cycle activity is represented or achieved by
i.a.5) the heterologous expression of ATP-dependent malate thiokinase and/or a malyl-CoA lyase.

It is preferred for the genetically modified cell according to the invention that the genetic modification of the cell provided in step i) comprises
b.1) the heterologous expression of one or more genes encoding glucose-1-phosphatase, glucose-6-phosphatase, alpha,alpha-trehalose phosphorylase, trehalase, Pase12, and/or Pase-MG7, and/or (preferably: and optionally),
b.2) a deletion of or a reduction of the expression of one or more genes encoding phosphoglycerate mutase, phosphopyruvate hydratase, phosphoglucomutase, glucose-1-phosphate uridylyltransferase, glucose-1-phosphate adenylyltransferase, and/or glucokinase.

Additionally or alternatively, it is preferred for the genetically modified cell according to the invention that the genetic modification of the cell provided in step i) comprises
b.1) the heterologous expression of one or more genes encoding Pase15, and/or MF2, and/or (preferably: and optionally),
b.2) a deletion of or a reduction of the expression of one or more genes encoding phosphoglycerate mutase, phosphopyruvate hydratase, and/or glucose-6-phosphate isomerase.

Additionally or alternatively, it is preferred for the genetically modified cell according to the invention that the genetic modification of the cell provided in step i) comprises
b.2) a deletion of or a reduction of the expression of one or more genes encoding phosphoglycerate mutase, phosphopyruvate hydratase, and/or glucose-1-phosphate adenylyltransferase.

Additionally or alternatively, it is preferred for the genetically modified cell according to the invention that the genetic modification of the cell provided in step i) comprises
b.2) a deletion of or a reduction of the expression of one or more genes encoding phosphoglycerate mutase, phosphopyruvate hydratase, 4-alpha-glucanotransferase, UTP glucose-1-phosphate uridylyltransferase, glucokinase, phosphoglucomutase, and/or glycogen phosphorylase.

It is further preferred that the genetic mutation of the genetically modified cell according to the invention further comprises
c) the heterologous expression of one or more genes encoding phosphoglycerate kinase, glyceraldehyde-3-phosphate dehydrogenase, fructose-bisphosphate aldolase, phosphoglycerate kinase, glucose-6-phosphate isomerase, phosphoglucomutase, UTP glucose-1-phosphate uridylyltransferase, trehalose 6-phosphate synthase, trehalose-6-phosphatase, glucose-6-phosphate isomerase, phosphoglucomutase, glucose-1-phosphate adenylyltransferase, glycogen synthase, 1,4-alpha-glucan branching enzyme,
preferably of at least phosphoglycerate kinase,
preferably of at least glyceraldehyde-3-phosphate dehydrogenase,
preferably of at least fructose-bisphosphate aldolase,
preferably of at least phosphoglycerate kinase,
preferably of at least glucose-6-phosphate isomerase,
preferably of at least phosphoglucomutase,
preferably of at least UTP glucose-1-phosphate uridylyltransferase,
preferably of at least trehalose 6-phosphate synthase,
preferably of at least trehalose-6-phosphatase,
preferably of at least glucose-6-phosphate isomerase,
preferably of at least phosphoglucomutase,
preferably of at least glucose-1-phosphate adenylyltransferase,
preferably of at least glycogen synthase,
preferably of at least 1,4-alpha-glucan branching enzyme.

The present invention further relates to the use of a genetically modified cell according to the invention for producing one or more carbohydrate(s), preferably in a method according to the invention,
wherein the, one, two, three or all carbohydrate(s) is/are selected from the group consisting of glucose, fructose, trehalose, and starch.

What was said herein with regard to the method according to the invention and/or with regard to the genetically modified cell according to the invention applies accordingly to the use according to the invention.

The present invention further relates to a mixture, preferably biomass, comprising
- the genetically modified cell according to the invention, and
- one or more carbohydrate(s), wherein the, one, two, three or all carbohydrate(s) is/are selected from the group consisting of glucose, fructose, trehalose, and starch.

Preferably, the term "biomass" refers to a mass of cells, preferably living cells, preferably non-living cells. Particularly preferably, the cells in the mixture or, respectively, biomass consist or essentially consist of the genetically modified cell according to the invention. Preferably, the term "essentially consists of" describes that at least 80 % of the cells, preferably at least 85 %, particularly preferably at least 90 %, further preferably at least 95 %, especially preferably at least 98 %, more preferably at least 99 % of the cells are a genetically modified cell according to the invention. The percentage of cells may be determined by typical methods in the art, e.g. by cell sorting. For this purpose, the genetically modified cell according to the invention may comprise a reporter, e.g. a reporter gene, e.g. a reporter gene encoding GFP.

Preferably, the total amount of the one or more carbohydrate(s) in the biomass, particularly preferably the summed amount of glucose, fructose, trehalose, and starch in the biomass, is at least 10 wt-% preferably at least 20 wt.-%, further preferably at least 30 wt.-%, particularly preferably at least 40 wt.-%, especially preferably at least 50 wt.-%, more preferably at least 60 wt.-%, even further preferably at least 70 wt.-%, based on the total weight of the biomass.

Preferably, the mixture is obtainable or obtained by a method according to the invention.

The genetic modification of the cell has been designed to obtain a sugary biomass, since the produced carbohydrate(s) essentially remain(s) in the cell. Particularly, the genetic modification may further prevent the cell to metabolize the produced carbohydrate(s) and the cells described herein lack transporters for e.g. glucose, fructose, starch, trehalose, sucrose and other carbohydrates, as described herein. Therefore, the produced carbohydrate(s) essentially remain(s) in the cell. This reduces or even eliminates the costs of downstream processes.

Thus, the mixture, preferably biomass, may be further used for several applications, e.g. as feed for animals or as feed or eco-friendly carbon source for other microorganisms. It is of particular advantage that the mixture, preferably biomass, has a high content of the carbohydrates as described herein, wherein at the same time, the carbon footprint is significantly lower than currently existing alternatives.

What was said herein with regard to the method according to the invention and/or with regard to the genetically modified cell according to the invention and/or with regard to the use according to the invention applies accordingly to the mixture according to the invention.

The present invention further relates to an animal feed preparation, cell culture medium or cell culture supplement, comprising the mixture, preferably biomass, according to the invention.

What was said herein with regard to the method according to the invention and/or with regard to the genetically modified cell according to the invention and/or with regard to the use according to the invention, and/or with regard to the mixture according to the invention, applies accordingly to the animal feed preparation, cell culture medium or cell culture supplement according to the invention.

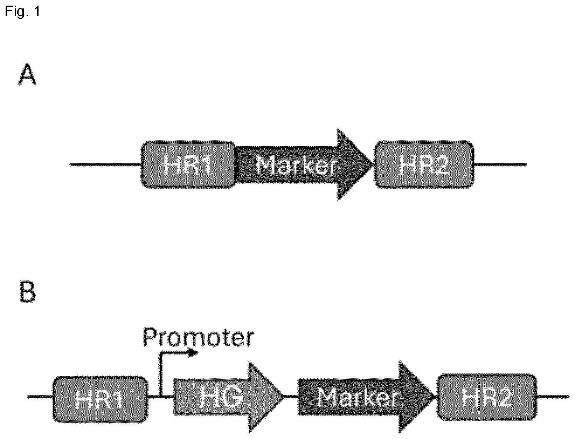

Fig 1. is a schematic diagram of the cassettes used for genome modification in the example section with HR = homologous region; HG = heterologous gene; and Marker = antibiotic marker. (A) the schematic diagram of the cassette used for gene deletion without marker recycling, and (B) the schematic diagram of the cassette used for the integration of heterologous gene. HR1 and HR2 were specific to the target locus for each transformation, Marker was the thermostable antibiotic marker used for the selection of genetically modified strains after transformation and genome integration of the cassette, and HG was the heterologous gene(s) used for the expression of a different gene based on the experiment requirement. Promoter stands for any promoter used in the experiments (constitutive or inducible).

Further aspects and advantages of the invention result from the subsequent description of preferred examples.

### Examples

### Example 1: Engineering HT that expresses heterologous proteins; green fluorescence protein:

To test the genetic modification capability of *Hydrogenobacter thermophilus* (HT), both plasmids-based, and genomic integration approaches were tested. Genomic integration approaches included methods where the antibiotic selection marker could or could not be recycled, as well as the suicide plasmid approach. To test whether these approaches work in HT, and to see if HT can express heterologous proteins, green fluorescence protein (GFP) which may or may not have been thermostable, was used. For this purpose, the GFP may be a synthetic GFP protein or from an organism that produces it. For generating the integration cassette for integrating a heterologous gene or for deleting a native gene of HT the cassette was designed as shown in Fig. 1. The cassette contains 1000 - 2000 bp of the upstream and downstream regions of the targeted gene locus. Between the two regions was a promoter (constitutive/ inducible) controlling the GFP expression, ORF of GFP, a terminator, an antibiotic marker, and other components depending on the need.

The GFP may or may not be codon optimized for the expression in the host-microbe. More than one genetic locus was used to test the integration and the expression of GFP. The loci were chosen both from the chromosome as well as the plasmid. The transformation was carried out using a standard transformation protocol. The transformants were grown on standardized growth medium containing agar and appropriate antibiotic(s). The transformants were grown heterotrophically (malate as carbon source) or autotrophically in CO₂, O₂ and H₂ atmospheres. Thermostable antibiotic markers were used for the selection of the transformants. The colonies appeared after 48-72 h of incubation at 52 °C. The colonies were tested by colony PCR to verify if they were transformed, and to verify if the expression cassette was inserted into the targeted locus. The GFP expression under the constitutive promoter(s) used in this study was easily seen under the blue light table. This success demonstrated that HT can be genetically modified and can express different heterologous proteins.

### Example 2: Deletion of one or more gene(s) in HT:

To test the genetic modification capability of *Hydrogenobacter thermophilus* (HT), the gene deletion in HT was also tested. This approach included methods where the antibiotic selection marker could or could not be recycled, as well as suicide plasmid approach. To test whether these approaches work in HT, the cassette was designed that contained the upstream and the downstream region also known as the homologous region, of the targeted locus that was planned to be deleted.

The length of the homologous region varies based on the microbes. For this experiment, the homologous region used was between 500-1500 bp. Fig. 1 shows the schematic diagram of the cassette. The cassette contains an antibiotic marker between the upstream and the downstream region of the targeted locus. More than one genetic locus was deleted using this approach. The largest region deleted was around 20,000 bp. The loci were chosen both from the chromosome as well as the plasmid. The transformation was carried out using a standard transformation protocol. The transformants were grown on a standardized growth medium containing agar and appropriate antibiotic(s). The transformants were grown heterotrophically (malate as carbon source) or autotrophically in CO₂, O₂ and H₂ atmospheres. Thermostable antibiotic markers were used for the selection of the transformants. The colonies appeared after 48-72 h of incubation at 52 °C. The colonies were tested by colony PCR to verify if they are transformed and to verify if the gene was deleted. With the help of a qRT-PCR, the expression of the deleted gene was measured to confirm the deletion of the corresponding gene. This success demonstrated that gene deletion is possible in HT.

### Example 3: Engineering HT to express heterologous enzymes and modifying the natural pathways to produce glucose

*Hydrogenobacter thermophilus* (HT) undergoes glycolysis to produce the precursors that are required by different pathways of the cell. Advantageously, the enzymes of the glycolysis pathway catalyse bidirectional reactions, with which it is possible to utilize the pathways as desired. By deleting enzymatic reactions that branch the glycolysis to other pathways, it is possible to direct the glycolysis pathway for glucose production, as described in Example 2.

Further heterologous enzymes that catalyze the conversion of glucose-6-phosphate or glucose-1-phosphate to glucose were chosen and expressed in HT, as described in Example 1. The enzymes may be from a eukaryote or a prokaryote, may or may not have been a thermophile and the gene coding for these enzymes may or may not have been codon optimized. Genomic integration approaches included methods where the antibiotic selection marker could or could not be recycled, as well as suicide plasmid approach.

For the genetic integration of the desired genes, the cassette used contained the following components, the upstream and the downstream region of the targeted locus (where the integration of the heterologous gene is intended), as exemplarily shown in Fig. 1. Between the two homologous regions was a promoter (constitutive/ inducible) controlling the gene expression, a terminator, an antibiotic marker and other components depending on the need. The gene may or may not be codon optimized for the expression in the host microbe. More than one genetic locus was used to test the integration and the expression of different genes involved in this pathway. The enzymes were overexpressed using (constitutive/inducible) promoters. The deletion of the desired gene to stop branching of this pathway and to increase the availability of glucose-6-phosphate and glucose-1-phosphate was carried out. The cassette used contained the homologous region of the gene/locus to be deleted and an antibiotic marker for the selection of the transformant.

The transformation was carried out using a standard transformation protocol. The transformants were grown on standardized growth medium containing agar and appropriate antibiotic(s). The transformants were grown heterotrophically (malate as carbon source) or autotrophically in CO₂, O₂ and H₂ atmosphere. Thermostable antibiotic markers were used for the selection of the transformants. The colonies appeared after 48-72 h of incubation at 52 °C. The colonies were tested by colony PCR to verify if they are transformed, and to verify if the expression cassette was inserted into the targeted locus.

The successfully genetically modified HT strain was grown autotrophically in synthetic designed medium in the parallel reactor system tested for the expression of glucose. Depending on the need, sugar production was triggered by the addition of an inducer in the growth medium. The increased production of glucose was confirmed by HPLC. This success demonstrated that modifying the natural pathways to produce glucose is possible in HT.

Advantageously, the cells did not need to be stressed to produce glucose and no special media requirements or growth conditions were required by the genetically modified HT.

### Example 4: Genetically engineering HT to express heterologous enzymes and to modify the natural pathways to produce fructose

*Hydrogenobacter thermophilus* (HT) is incapable of utilizing fructose as a carbon source, it lacks fructokinase that catalyzes the conversion of D-Fructose to Fructose-6P. However, HT contains enzymes that catalyze the conversion of D-Glucose-6P to D-Fructose-6P. Heterologous enzymes that could perform the removal of phosphate from D-Fructose-6P were chosen and tested. The enzyme could have been from a eukaryote or a prokaryote, may or may not have been a thermophile and the gene coding for these enzymes may or may not be codon optimized. The enzyme could have also been synthetically designed or a modified enzyme for another microbe. Genomic integration approaches included methods where the antibiotic selection marker could or could not be recycled, as well as suicide plasmid approach.

For the genetic integration of the desired gene, the cassette used contained the following components, the upstream and the downstream region of the targeted locus. Between the two regions was a promoter (constitutive/ inducible) controlling gene expression, a terminator, an antibiotic marker and other components depending on the need. These enzymes were overexpressed using promoters (constitutive/inducible). The transformation was carried out using a standard transformation protocol. The transformants were grown on standardized growth medium containing agar and appropriate antibiotic(s). The transformants were grown heterotrophically (malate as carbon source) or autotrophically in CO₂, O₂ and H₂ atmospheres.

Thermostable antibiotic markers were used for the selection of the transformants. The colonies appeared after 48-72 h of incubation at 52 °C. The colonies were tested by colony PCR to verify if they were transformed and to verify if the expression cassette was inserted into the targeted locus. qRT-PCR was performed to determine the expression of the heterologous gene (enzyme).

The successfully genetically modified HT strain was grown autotrophically in the synthetically designed medium in the parallel reactor system. By testing the samples with HPLC, the fructose production in the genetically modified microbe could be confirmed.

Advantageously, the cells did not need to be stressed to produce fructose and no special media requirements or growth conditions were required by the genetically modified HT.

### Example 5: Genetically engineering HT for producing Trehalose

*Hydrogenobacter thermophilus* (HT) has the enzymes that are required to produce trehalose. The genes involved in the trehalose production, as described above, are present in a single operon.

The promoter of said operon was replaced by a strong constitutive promoter or an inducible promoter, to control the expression of the genes. This was achieved by inserting an inducible heterologous or homologous promoter of HT. The insertion of the promoter is achieved by replacing the original promoter with the help of double homologous recombination with or without marker recycling, as described in examples 1 to 4.

Further, phosphopyruvate hydratase and glucose-1-phosphate adenylyltransferase were deleted in HT with the method as described in Example 2.

The successfully genetically modified HT strain was grown autotrophically in the synthetically designed medium in the parallel reactor system. By testing the samples with HPLC, the increased trehalose production in the genetically modified microbe could be confirmed.

Advantageously, the cells did not need to be stressed to produce trehalose and no special media requirements or growth conditions were required by the genetically modified HT (unlike the wild-type HT).

### Example 6: Genetically engineering HT to produce Starch

*Hydrogenobacter thermophilus* (HT) has the genes that are required for the synthesis/production of starch. The genes involved in the starch production, as described above, are present in a single operon.

The promoter of said operon was replaced by a strong constitutive promoter or an inducible promoter, to control the expression of the genes. This was achieved by inserting an inducible heterologous or homologous promoter of HT. The insertion of the promoter is achieved by replacing the original promoter with the help of double homologous recombination with or without marker recycling, as described in examples 1 to 4.

Further, UTP-glucose-1-phosphate uridylyltransferase, which prevents the conversion of glucose-1P to UDP-glucose, 4-alpha-glucanotransferase to prevent the degradation of starch to glucose-1-phosphate, phosphoglycerate mutase, phosphoglucomutase, and/or glucokinase were deleted in HT with the method as described in Example 2.

The successfully genetically modified HT strain was grown autotrophically in the synthetically designed medium in the parallel reactor system. By testing the samples with HPLC, the increased starch production in the genetically modified microbe could be confirmed.

Advantageously, the cells did not need to be stressed to produce starch and no special media requirements or growth conditions were required by the genetically modified HT (unlike the wild-type HT).

## Claims

1. Method for producing one or more carbohydrate(s),
wherein the, one, two, three or all carbohydrate(s) is/are selected from the group consisting of glucose, fructose, trehalose, and starch,
the method comprising the steps
i) providing a genetically modified cell,
wherein the cell is selected from the genus *Hydrogenophilus,* preferably of the species *Hydrogenophilus thermoluteolus,*
wherein the genetic modification is or comprises
a) a reduction of the Calvin-Benson cycle activity and/or a reversion of the glyoxylate cycle activity, and/or an introduction of the reductive TCA cycle activity,
and
b.1) the heterologous expression of one, two, three or more or all gene(s) encoding an enzyme of group A,
wherein the enzymes of group A are selected from the group consisting of glucose-1-phosphatase, glucose-6-phosphatase, alpha,alpha-trehalose phosphorylase, trehalase, Pase12, Pase-MG7, Pase15, MF2, and combinations thereof,
and/or
b.2) a deletion of or a reduction of the expression of one, two, three or more or all gene(s) encoding an enzyme of group B,
wherein the enzymes of group B are selected from the group consisting of phosphopyruvate hydratase, phosphoglucomutase, glucose-1-phosphate uridylyltransferase, glucose-1-phosphate adenylyltransferase, glucose-6-phosphate isomerase, glycogen phosphorylase, glucokinase, 4-alpha-glucanotransferase, UTP glucose-1-phosphate uridylyltransferase, phosphoglycerate mutase, and combinations thereof,
ii) cultivating the cell provided in step i) at a temperature in the range of from 40 to 65 °C, preferably of from 42.5 to 62.5 °C, particularly preferably of from 45 to 60 °C, further preferably of from 47.5 to 57.5 °C, more preferably of from 50 to 55 °C,
wherein cultivating includes providing CO₂, O₂ and H₂,
iii) purifying the produced carbohydrate(s) from the cell or cell culture cultivated in step ii).

2. Method according to claim 1, wherein the reduction of the Calvin-Benson cycle activity is represented or achieved by
i.a.1) the deletion or a reduction of the homologous expression of ribulose-1,5-bisphosphat-carboxylase/-oxygenase,
and/or
wherein the introduction of the reductive TCA cycle activity is represented or achieved by
i.a.1) the deletion or a reduction of the homologous expression of ribulose-1,5-bisphosphat-carboxylase/-oxygenase, and/or
i.a.2) an increase of the expression of citrate synthase, and/or
i.a.3) the heterologous expression of citryl-CoA synthetase (CCS) and/or citryl-CoA lyase (CCL), preferably citryl-CoA synthetase (CCS) and/or citryl-CoA lyase (CCL) from *Hydrogenobacter thermophilus,* and/or
i.a.4) the heterologous expression of pyruvate carboxylase and/or pyruvate synthase and/or alpha-ketoglutarate synthase, preferably pyruvate carboxylase and/or pyruvate synthase and/or alpha-ketoglutarate synthase from *Hydrogenobacter thermophilus,* and/or
i.a.5) the heterologous expression of ATP-dependent malate thiokinase and/or a malyl-CoA lyase,
and/or
wherein the reversion of the glyoxylate cycle activity is represented or achieved by
i.a.5) the heterologous expression of ATP-dependent malate thiokinase and/or a malyl-CoA lyase.

3. Method according to any of the preceding claims, wherein the cultivation in step ii) is applied for at least 2 h, preferably for at least 4 h, particularly preferably for at least 6 h, further preferably for at least 8 h.

4. Method according to any of the preceding claims, wherein the genetic modification of the cell provided in step i) comprises
- b.1) the heterologous expression of one or more genes encoding glucose-1-phosphatase, glucose-6-phosphatase, alpha,alpha-trehalose phosphorylase, trehalase, Pase12, and/or Pase-MG7, and/or
b.2) a deletion of or a reduction of the expression of one or more genes encoding phosphoglycerate mutase, phosphopyruvate hydratase, phosphoglucomutase, glucose-1-phosphate uridylyltransferase, glucose-1-phosphate adenylyltransferase, and/or glucokinase
and/or
- b.1) the heterologous expression of one or more genes encoding Pase15, and/or MF2, and/or
b.2) a deletion of or a reduction of the expression of one or more genes encoding phosphoglycerate mutase, phosphopyruvate hydratase, and/or glucose-6-phosphate isomerase,
and/or
- b.2) a deletion of or a reduction of the expression of one or more genes encoding phosphoglycerate mutase, phosphopyruvate hydratase, and/or glucose-1-phosphate adenylyltransferase,
and/or
- b.2) a deletion of or a reduction of the expression of one or more genes encoding phosphoglycerate mutase, phosphopyruvate hydratase, 4-alpha-glucanotransferase, UTP glucose-1-phosphate uridylyltransferase, glucokinase, phosphoglucomutase, and/or glycogen phosphorylase.

5. Method according to any of the preceding claims, wherein the genetic mutation of the cell provided in step i) further comprises
c) the heterologous expression of one or more genes encoding phosphoglycerate kinase, glyceraldehyde-3-phosphate dehydrogenase, fructose-bisphosphate aldolase, phosphoglycerate kinase, glucose-6-phosphate isomerase, phosphoglucomutase, UTP glucose-1-phosphate uridylyltransferase, trehalose 6-phosphate synthase, trehalose-6-phosphatase, glucose-6-phosphate isomerase, phosphoglucomutase, glucose-1-phosphate adenylyltransferase, glycogen synthase, 1,4-alpha-glucan branching enzyme.

6. Genetically modified cell,
wherein the cell is selected from the genus *Hydrogenophilus,* preferably of the species *Hydrogenophilus thermoluteolus,*
wherein the genetic modification is or comprises
a) a reduction of the Calvin-Benson cycle activity and/or a reversion of the glyoxylate cycle activity, and/or an the introduction of the reductive TCA cycle activity,
and
b.1) the heterologous expression of one, two, three or more or all gene(s) encoding an enzyme of group A,
wherein the enzymes of group A are selected from the group consisting of glucose-1-phosphatase, glucose-6-phosphatase, alpha,alpha-trehalose phosphorylase, trehalase, Pase12, Pase-MG7, Pase15, MF2, and combinations thereof, and/or
b.2) a deletion of or a reduction of the expression of one, two, three or more or all gene(s) encoding an enzyme of group B,
wherein the enzymes of group B are selected from the group consisting of phosphopyruvate hydratase, phosphoglucomutase, glucose-1-phosphate uridylyltransferase, glucose-1-phosphate adenylyltransferase, glucose-6-phosphate isomerase, glycogen phosphorylase, glucokinase, 4-alpha-glucanotransferase, UTP glucose-1-phosphate uridylyltransferase, phosphoglycerate mutase, and combinations thereof.

7. Genetically modified cell according to claim 6, wherein the reduction of the Calvin-Benson cycle activity is represented or achieved by
a.1) the deletion or a reduction of the homologous expression of ribulose-1,5-bisphosphat-carboxylase/-oxygenase,
and/or
wherein the introduction of the reductive TCA cycle activity is represented or achieved by
i.a.1) the deletion or a reduction of the homologous expression of ribulose-1,5-bisphosphat-carboxylase/-oxygenase, and/or
i.a.2) an increase of the expression of citrate synthase, and/or
i.a.3) the heterologous expression of citryl-CoA synthetase (CCS) and/or citryl-CoA lyase (CCL), preferably citryl-CoA synthetase (CCS) and/or citryl-CoA lyase (CCL) from *Hydrogenobacter thermophilus,* and/or
i.a.4) the heterologous expression of pyruvate carboxylase and/or pyruvate synthase and/or alpha-ketoglutarate synthase, preferably pyruvate carboxylase and/or pyruvate synthase and/or alpha-ketoglutarate synthase from *Hydrogenobacter thermophilus,* and/or
a.5) the heterologous expression of ATP-dependent malate thiokinase and/or a malyl-CoA lyase,
and/or
wherein the reversion of the glyoxylate cycle activity is represented or achieved by
a.5) the heterologous expression of ATP-dependent malate thiokinase and/or a malyl-CoA lyase.

8. Genetically modified cell according to any of claims 6 or 7, wherein the genetic modification comprises
- b.1) the heterologous expression of one or more genes encoding glucose-1-phosphatase, glucose-6-phosphatase, alpha,alpha-trehalose phosphorylase, trehalase, Pase12, and/or Pase-MG7, and/or
b.2) a deletion of or a reduction of the expression of one or more genes encoding phosphoglycerate mutase, phosphopyruvate hydratase, phosphoglucomutase, glucose-1-phosphate uridylyltransferase, glucose-1-phosphate adenylyltransferase, and/or glucokinase
and/or
- b.1) the heterologous expression of one or more genes encoding Pase15, and/or MF2, and/or
b.2) a deletion of or a reduction of the expression of one or more genes encoding phosphoglycerate mutase, phosphopyruvate hydratase, and/or glucose-6-phosphate isomerase,
and/or
- b.2) a deletion of or a reduction of the expression of one or more genes encoding phosphoglycerate mutase, phosphopyruvate hydratase, and/or glucose-1-phosphate adenylyltransferase,
and/or
- b.2) a deletion of or a reduction of the expression of one or more genes encoding phosphoglycerate mutase, phosphopyruvate hydratase, 4-alpha-glucanotransferase, UTP glucose-1-phosphate uridylyltransferase, glucokinase, phosphoglucomutase, and/or glycogen phosphorylase.

9. Genetically modified cell according to any of claims 6 to 8, wherein the genetic mutation of the genetically modified cell according to the invention further comprises
c) the heterologous expression of one or more genes encoding phosphoglycerate kinase, glyceraldehyde-3-phosphate dehydrogenase, fructose-bisphosphate aldolase, phosphoglycerate kinase, glucose-6-phosphate isomerase, phosphoglucomutase, UTP glucose-1-phosphate uridylyltransferase, trehalose 6-phosphate synthase, trehalose-6-phosphatase, glucose-6-phosphate isomerase, phosphoglucomutase, glucose-1-phosphate adenylyltransferase, glycogen synthase, 1,4-alpha-glucan branching enzyme.

10. Use of a genetically modified cell according to any of claims 6 to 9 for producing one or more carbohydrate(s), preferably in a method according to any of claims 1 to 5,
wherein the, one, two, three or all carbohydrate(s) is/are selected from the group consisting of glucose, fructose, trehalose, and starch.

11. Mixture, preferably biomass, comprising
- the genetically modified cell according to any of claims 6 to 9, and
- one or more carbohydrate(s), wherein the, one, two, three or all carbohydrate(s) is/are selected from the group consisting of glucose, fructose, trehalose, and starch.

12. Mixture according to claim 11, wherein the mixture is obtainable or obtained by a method according to any of claims 1 to 5.

13. Animal feed preparation, cell culture medium or cell culture supplement, comprising the mixture, preferably biomass, according to claims 11 or 12.
